# EUROPÄISCHE PATENTSCHRIFT

(11) **EP 2 244 693 B1**
(45) Veröffentlichungstag und Bekanntmachung des Hinweises auf die Patenterteilung: **16.05.2012**
(21) Anmeldenummer: 08701149.0
(22) Anmeldetag: 18.01.2008
(51) Int. Cl.: A61K 9/00, A61K 31/555, A61K 9/127, A61K 47/22, A61K 47/24

(54) **MITTEL ZUR INTRAARTIKULÄREN INJEKTION**
AGENT FOR INTRA-ARTICULAR INJECTION
PRODUITS POUR INJECTION INTRA-ARTICULAIRE

(43) Veröffentlichungstag der Anmeldung: 03.11.2010
(73) Patentinhaber: Kief, Horst, 67069 Ludwigshafen (DE)
(72) Erfinder: Kief, Horst, 67069 Ludwigshafen (DE)
(74) Vertreter: Wagner, Jutta
(86) Internationale Anmeldenummer: PCT/EP2008/000365
(87) Internationale Veröffentlichungsnummer: WO 2009/089845

(56) Entgegenhaltungen:
- WO-A-2006/039336
- WO-A-2006/131737
- US-A1- 2002 068 718
- US-A1- 2006 122 147

## Beschreibung

Die vorliegende Erfindung betrifft Arzeneimittel enthaltend Vitamin E zur intraartikulären Inkjektion.

Die Wirksamkeit von Vitamin E bei Erkrankungen des rheumatischen Formenkreises kann als gesichert gelten. Dazu werden relativ hohe Dosen von 400 mg - 1000 mg sowohl enteral als auch parenteral appliziert. Den therapeutischen Wert eines zumindest teilweise wasserlöslichen Vitamin E's hat man erkannt und die Wasserlöslichkeit des Vitamins verbessert durch Acetatbildung. Jedoch ist die Wasserlöslichkeit dadurch nur graduell mehr im Sinne einer Suspensin verbessert, sodass beispielsweise das Alpha-Tocopherol in seiner Acetatform nur intramuskulär gegeben werden darf. Eine intravenöse Anwendung verbietet sich daher.

Eine interessante, therapeutische Möglichkeit der Anwendung von Vitamin E wäre die Anwendung intraartikulär, beispielsweise bei Gelenkarthrosen. Hier hat das Vitamin E aber den Nachteil, dass durch die Kommunikation der Gelenkhöhle mit dem Gefäßsystem die Gefahr der Fettembolie besteht, weshalb bei einschlägigen Präparaten die intraartikuläre Injektion ausdrücklich kontraindiziert ist.

Die US 2006/0122147 beschreibt Hyaluronsäure-Zusammensetzungen, die u.a. auch Tocopherol, Chondroitinsulfat oder Glucocorticoide enthalten können. Die Applikation soll auf allen bekannten Routen möglich sein.

US 2002/0068718 befasst sich ebenfalls mit Hyaluronsäure. Vitamin E und Chondroitinsulfat werden als mögliche weitere Inhaltsstoffe bneben einer Vielzahl anderer Substanzen erwähnt. Die Zusammensetzungen sollen oral oder topisch appliziert werden.

Die WO 2006/031737 beschreibt Zusammensetzungen zur Behandlung von entzündlichen Erkrankungen, im Vordergrund steht Heuschnupfen. Die zur nasalen Anwendung konzipierten Zusammensetzungen sollen Liposomen auf Basis polarer Lipide und entzündungshemmende Wirkstoffe enthalten. Zu den Wirkstoffen gehören Glukokortikoide, als polare Lipide sind u.a. Phospholipide genannt.

WO 2006/039336 betrifft Retard-Zusammensetzungen. Es wird eine Vielzahl an Wirkstoffen genannt, auch allgemein auf Kombinationen hingewiesen. In den Beispielen finden sich auch Zusammensetzunge enthaltend Vitamin E und Dexamethason.

Für keine der Zusammensetzungen wird eine spezifische Wirkung bzw. Verträglichkeit bei Erkrankungen des rheumatischen Formenkreises mit klinischen Befunden belegt.

Die geschilderten Nachteile von Vitamin E werden durch die vorzuschlagende Neuerung beseitigt. Dazu wird das Alpha-Tocopherol, auch in seiner Acetatform, mit einem Phospholipid gemischt. Die Zusammensetzung der Phospholipide, Ceramide, Encephaline oder Lecithin kann variabel sein, vorzugsweise wird jedoch Phosphatidycholin eingesetzt. Das ideale Mischungsverhältnis von Alpha-Tocopherol zu Phospholipid beträgt dabei von 1:1 bis 1:2,5, vorzugsweise 1:2.

Bei der Einbringung in Gelenke hat das Alpha-Tocopherol-Phospholipidgemisch den Vorteil der verbesserten Viskosität, sodass die Mischbarkeit mit Lokalanästhetika wesentlich erleichtert ist und durch die verbesserte Viskosität die Kontamination des gesamten Gelenkraumes gewährleistet ist. Für die Phospholipide, speziell Phosphatidylcholin, ist eine sehr gute Gewebsgängigkeit nachgewiesen, sodass im Zuge des Schleuseffektes dieser Substanz das Alpha-Tocopherol seinen membranstabilisierenden oder auch antientzündlichen Effekt im zu behandelnden Areal rascher und besser erfüllen kann.

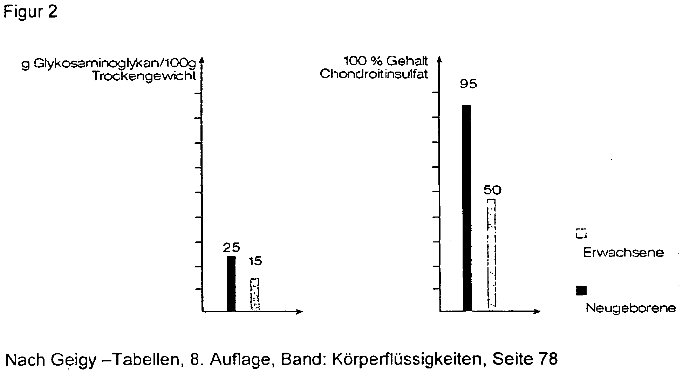

Speziell im Bereich der intraartikulären Injektion ist die Wirksamkeit von Proteoglycanen nachgewiesen. Dazu stehen mehrere Präparate, die aus Hyaluronsäure bestehen, auf dem Arzneimittelmarkt zur Verfügung. Seltsamerweise wird Chondroitinsulfat, ein Proteoglycan, das aus der Gruppe der gelenkeigenen Proteoglycane herausragt, da es besonders in frühester Jugend in höherem Maße im gelenkeigenen Knorpel vorhanden ist, vergleiche Figur 2, nach entsprechender arzneimittelgerechter Aufbereitung als intraartikuläre Injektion bislang nicht eingesetzt.

Ein Gemisch aus 50 mg Tocopherolacetat, 150 mg Phosphatidylcholin und 100 mg Chondroitinsulfat, das aus Haifischknorpel gewonnen wurde und nach arzneimittelrechtlichen und pharmazeutischen Gesichtspunkten aufbereitet wurde, führt erfindungsgemäß in den weitaus häufigsten Fällen zu einer deutlichen Verbesserung der Beschwerden bei Gelenkarthrosen, da die heilenden Wirkungen der Einzelsubstanzen sich in der Kombination offensichtlich potenzieren.

Überraschenderweise stellte sich heraus, dass das beschriebene Gemisch durch Beimengung von Folsäure - im vorliegenden Fall z.B. 10 mg Folsäure in wässriger Lösung - noch weiter homogenisiert wird. Die Lösung bleibt selbst bei monatelanger, kühler Lagerung vollständig klar.

Darüber hinaus zeigt sich diese Lösung aufnahmefähig für wässrige Diclophenaclösung. Diclophenac wird üblicherweise nicht intraartikulär appliziert, kann jedoch in der beschriebenen Mischung nicht nur vollständig gefahrlos appliziert werden, sondern führt durch seinen antiphlogistischen Effekt darüber hinaus noch zu einer deutlich besseren Verträglichkeit der applizierten Mischung.

Bei aktivierten Arthrosen ist es sinnvoll, die beschriebene Medikamentenkombination mit Cortisonpräparaten zu mischen. Überraschenderweise stellte sich dabei heraus, dass Kristallsuspensionen aus Dexamethasonacetat, die der beschriebenen Kombination untergemischt wurden, bis zu zwei Jahre Beschwerdefreiheit nach sich zogen. Die Anwendung reiner Kristallsuspensionen in das erkrankte Gelenk ist umstritten, da man den Kristallen einen zusätzlichen mechanischen Verschleiß der Knorpeloberfläche zuschreibt. In der Tat halten die schmerzlindernden und antientzündlichen Effekte derartiger Injektionen in der Regel nur wenige Tage oder Wochen.

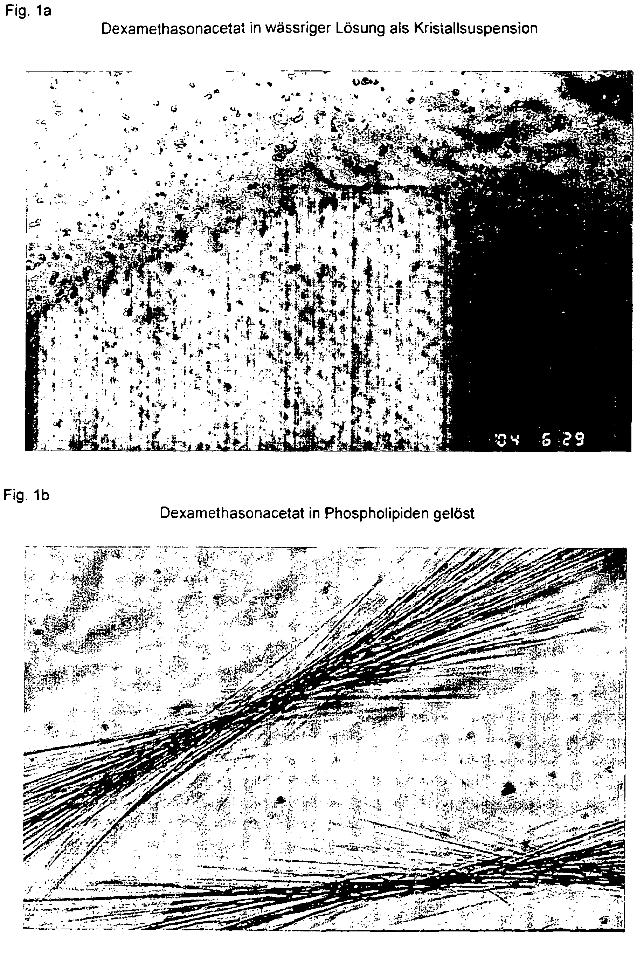

Aufgrund der ganz andersartigen klinischen Ergebnisse mittels der geschilderten Kombination wurden die Dexamethasonkristalle in reiner Kochsalzlösung und in der beschriebenen Neuerung mikroskopisch untersucht. Danach waren die Kristalle in Phospholipidlösung innerhalb von sechs Stunden um 50 % vermindert und nach zwölf Stunden nicht mehr nachweisbar. Dagegen zeigten sich sehr dünne, nadelförmige Formationen, die von ihrer Struktur her offensichtlich nicht in der Lage sind, den Knorpel mechanisch zu schädigen (siehe Figuren 1a und 1b). Die beschriebene Medikamentenkombination ist demnach in der Lage, durch Umlagerung der Dexamethasonmoleküle offenbar eine physiologische Depotwirkung zu erzielen, die bislang unbekannt war. Die Kontrolle mit HPLC ergab sodann den Nachweis der strangförmigen Polymerisate als Dexamethasonacetat.

Die Neuerung zeigt außerdem den Vorteil, dass aufgrund der höheren Effektivität weniger Injektionen pro Gelenk notwendig sind. Darüber hinaus hält die schmerzlindernde Wirkung bis zu 2 Jahre an.

Zur Reduzierung der Reibung degenerativ veränderter Gelenkflächen aufeinander ist kein Mittel besser geeignet als Öl. Eine direkte Injektion von Öl in das erkrankte Gelenk ist jedoch aus den eingangs aufgeführten Gründen (Gefahr einer Fettembolie) nicht ungefährlich.

Prinzipiell würden sich verschiedene Öle für diesen Zweck eignen, die bereits zur Erzielung einer Depotwirkung Medikamenten beigemischt werden. In physikalischer Hinsicht ist jedoch Rhizinusöl zur Verminderung der Scherwirkung optimal. Um Rhizinusöl ungefährlich unterzubringen, wird daher die Bildung von Liposomen vorgeschlagen. Das vorgeschlagene Phospholipid eignet sich hervorragend zur Bildung von Rhizinusölliposomen. Liposomenbildung auf Phospholipidbasis ist Stand der Technik. Unbekannt ist jedoch die Anwendung von Liposomen zur intraartikulären Injektion, insbesondere mit den genannten Bestandteilen.

Obwohl Rhizinusöl ein hochvisköses Öl ist, lässt es sich in der in der Neuerung geschilderten Aufbereitung durch die feinsten im Handel erhältlichen Kanülen injizieren. Damit wird die Anwendung auch in kleinen Gelenken, bspw. Fingergelenken, problemlos möglich.

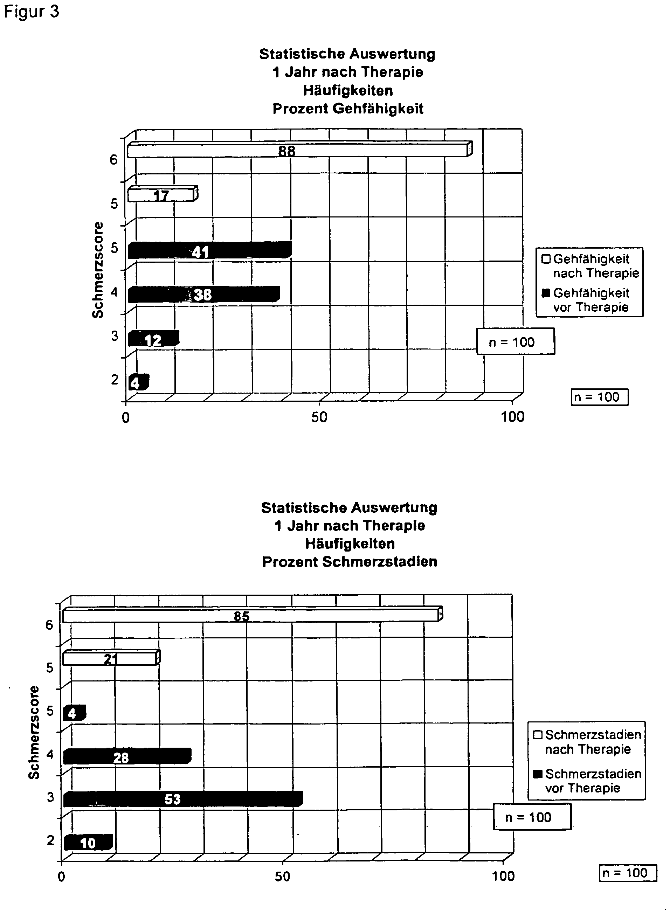

Die Ergebnisse einer derartigen Behandlung mit 1 - 2 Injektionen pro Gelenk zeigt die Statistik in Figur 3 in der 100 Patienten, der überwiegende Anteil mit Knie- und Hüftgelenksarthrosen, erfasst wurden. Der beschwerdefreie Intervall ist vergleichsweise lang, im Standardfall etwa 12 Monate.

## Patentansprüche

1. Mittel zur intraartikulären Injektion, **dadurch gekennzeichnet, dass** es ein Gemisch von Alpha-Tocopherol, Phospholipiden, Proteoglykanen und eine Cortisonkristallsuspension oder eine Cortisonkristalllösung enthält.

2. Mittel nach Anspruch 1, **dadurch gekennzeichnet, dass** es als Proteoglykan Chondroitinsulfat enthält.

3. Mittel nach Anspruch 1 oder 2, **dadurch gekennzeichnet, dass** das Mischungsverhältnis von Alpha-Tocopherol zu Phospholipiden von 1:1 bis 1:2,5 beträgt.

4. Mittel nach mindestens einem der Ansprüche 1 - 3, **dadurch gekennzeichnet, dass** das Phospholipid Phosphatidylcholin ist.

5. Mittel nach mindestens einem der Ansprüche 1 - 4, **dadurch gekennzeichnet, dass** die Cortisonkristalllösung aus Dexamethasonacetat in Phospholipiden besteht.

6. Mittel nach mindestens einem der Ansprüche 1 - 5, **dadurch gekennzeichnet, dass** es 25 bis 75 mg Diclophenac in wässriger Lösung enthält.

7. Mittel nach mindestens einem der Ansprüche 1 - 6, **dadurch gekennzeichnet, dass** es 5 bis 15 mg Folsäure enthält.

8. Mittel nach mindestens einem der Ansprüche 1 - 7, **dadurch gekennzeichnet, dass** es durch Zugabe eines arzneimittelverträglichen Öls zum Phospholipid gebildete Liposomen enthält.

9. Mittel nach Anspruch 8, **dadurch gekennzeichnet, dass** das Öl Rizinusöl ist.

10. Mittel gemäß mindestens einem der Ansprüche 1 bis 9 zur Behandlung von Erkrankungen des rheumatischen Formenkreises, insbesondere von Arthrose, und ganz besonders von Gelenkarthrose.

## Claims

1. Agent for intra-articular injection, **characterized in that** it contains a mixture of alpha-tocopherol, phospholipids, proteoglycans, and a cortisone crystal suspension or a cortisone crystal solution.

2. Agent according to claim 1, **characterized in that** it contains chondroitin sulfate as proteoglycan.

3. Agent according to claim 1 or 2, **characterized in that** the mixing ratio of alpha-tocopherol to phospholipids is from 1:1 to 1:2.5.

4. Agent according to at least one of the claims 1 - 3, **characterized in that** the phospholipid is phosphatidylcholine.

5. Agent according to at least one of the claims 1 - 4, **characterized in that** the cortisone crystal solution consists of dexamethasone acetate in phospholipids.

6. Agent according to at least one of the claims 1 - 5, **characterized in that** it contains 25 to 75 mg diclofenac in aqueous solution.

7. Agent according to at least one of the claims 1 - 6, **characterized in that** it contains 5 to 15 mg folic acid.

8. Agent according to at least one of the claims 1 - 7, **characterized in that** it contains liposomes that are formed by adding a medicinal agent-compatible oil to the phospholipid.

9. Agent according to claim 8, **characterized in that** the oil is castor oil.

10. Agent according to at least one of the claims 1 to 9 for the treatment of
forms of rheumatic disease, in particular arthrosis, and even more particularly osteoarthritis.

## Revendications

1. Agent pour l'injection intraarticulaire, **caractérisé en ce qu'**il contient un mélange d'alpha-tocophérol, de phospholipides, de protéoglycanes et une suspension cristalline de cortisone ou une solution cristalline de cortisone.

2. Agent selon la revendication 1, **caractérisé en ce qu'**il contient du sulfate de chondroïtine à titre de protéoglycane.

3. Agent selon la revendication 1 ou 2, **caractérisé en ce que** le rapport de mélange de l'alpha-tocophérol aux phospholipides s'élève de 1:1 à 1:2,5.

4. Agent selon au moins une des revendications 1 à 3, **caractérisé en ce que** le phospholipide est la phosphatidylcholine.

5. Agent selon au moins une des revendications 1 à 4, **caractérisé en ce que** la solution cristalline de cortisone est constituée d'acétate de dexaméthasone dans des phospholipides.

6. Agent selon au moins une des revendications 1 à 5, **caractérisé en ce qu'**il contient de 25 à 75 mg de diclofenac en solution aqueuse.

7. Agent selon au moins une des revendications 1 à 6, **caractérisé en ce qu'**il contient de 5 à 15 mg d'acide folique.

8. Agent selon au moins une des revendications 1 à 7, **caractérisé en ce qu'**il contient des liposomes formés par addition au phospholipide d'une huile acceptable pharmaceutiquement.

9. Agent selon la revendication 8, **caractérisé en ce que** l'huile est de l'huile de ricin.

10. Agent selon au moins une des revendications 1 à 9, pour le traitement de maladies incluses dans la catégorie des maladies rhumatoïdes, en particulier de l'arthrose, et de manière tout à fait particulière, de lésions articulaires dégénératives.
